# EUROPÄISCHE PATENTSCHRIFT

(11) **EP 0 769 291 B1**
(45) Veröffentlichungstag und Bekanntmachung des Hinweises auf die Patenterteilung: **12.03.2003**
(21) Anmeldenummer: 96115526.4
(22) Anmeldetag: 27.09.1996
(51) Int. Cl.: A61K 7/48, A61K 33/30

(54) **Gegen Akne und entzündete Comedonen wirksame Wirkstoffkombinationen**
Active agent combination against acne and inflamed comedones
Combinaison d'agents actifs contre l'acné et les comédons enflammés

(30) Priorität: 17.10.1995 DE 19538555
(43) Veröffentlichungstag der Anmeldung: 23.04.1997
(73) Patentinhaber: Beiersdorf Aktiengesellschaft, 20245 Hamburg (DE)
(72) Erfinder: Schönrock, Uwe, Dr., 23866 Nahe (DE); Christiansen, Michael, 25436 Tornesch (DE); Steinke, Sigrid, 21075 Hamburg (DE); Untiedt, Sven, Dr., 20259 Hamburg (DE)

(56) Entgegenhaltungen:
- WO-A-93/21899
- DE-A- 4 232 143
- US-A- 5 445 823
- COSMETICS AND TOILETRIES, Bd. 103, Oktober 1988, Seiten 79-84, XP000617071 H. WESER ET AL: "Acceleration of Superficial Wound Healing By Panthenol Zinc Oxide"

## Beschreibung

Die vorliegende Erfindung betrifft Wirkstoffe und Zubereitungen, solche Wirkstoffe enthaltend, welche gegen Akne und entzündete Comedonen wirksam sind.

Bei der unreinen Haut sind neben anderen Einflüssen bakterielle Sekundärinfektionen von ätiologischer Bedeutung. Einer der wichtigsten Mikroorganismen, der in Zusammenhang mit unreiner Haut steht, ist Propionibacterium acnes.

Unreine Haut und/oder Komedonen beeinträchtigen das Wohlbefinden der Betroffenen aber selbst in leichten Fällen. Da praktisch jeder oder jede Jugendliche von unreiner Haut irgendeiner Ausprägung betroffen ist, besteht bei vielen Personen Bedarf, diesem Zustande abzuhelfen.

Beim Vollbilde der Akne, aber auch bei leichteren Ausprägungen, sind Entzündungen der Aknepusteln häufige Folge. Der Stand der Technik ließ es an Wirkstoffen, die eine befriedigende Behandlung im Sinne einer Heilung, aber auch einer Camouflage, also einer kosmetischen Abdeckung des Comedonen, mangeln

Aufgabe der vorliegenden Erfindung war es also, eine gegen Akne und entzündete Comedonen wirksame Stoffkombination zu finden.

Es hat sich überraschenderweise gezeigt, und darin liegt die Lösung der Aufgabe, daß die Verwendung kosmetischer und/oder dermatologischer Wirkstoffkombinationen gemäß Anspruch 1 gegen Akne und entzündete Comedonen, den Mißständen des Standes der Technik abhelfen.

Panthenol, chemisch: 2,4-Dihydroxy-N-(3-hydroxypropyl)-3,3-dimethylbutyramid, wird bekanntermaßen auf dem Gebiete der Kosmetik , aber auch zur Behandlung von Entzündungen eingesetzt. Es ist durch die Strukturformel 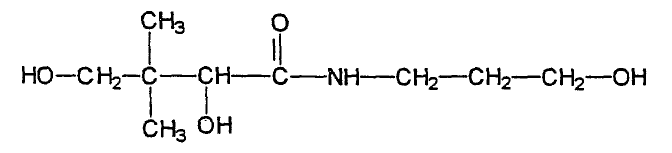 gekennzeichnet.

Zwar wird in Cosmetics & Toiletries Vol. 103, Oktober 1988, S. 79 ff. die Beschleunigung der Heilung von Wunden der Hautoberfläche beschrieben, die erfindungsgemäße Verwendung wird dadurch jedoch nicht nahegelegt. Bekanntlich haben Mittel, welche an sich in der Wundheilung Verwendung finden, bei der Aknebehandlung bestenfalls eine zufällig eintretende Wirkung. Ein Zusammenhang findet sich nicht

Ganz besonders vorteilhaft können die erfindungsgemäß verwendeten Wirkstoffkombinationen bei der Behandlung und Camouflage entzündeter Comedonen Anwendung finden.

Ferner ist aus DE-A-42 32 143 bekannt, Zinkoxid in Konzentrationen von 0,5 - 15 Gew.-% zur Behandlung von pustulösen Akneformen einzusetzen. Aus US-A-5 445 823 und WO 93/21899 ist die Verwendung von Panthenol als Antiirritans in Zubereitungen zur Behandlung von Akne bekannt, wobei diese Zubereitungen solche auf Basis Benzoylperoxid bzw. Salicylsäure sind.

Erfindungsgemäß können als günstige, aber dennoch fakultativ zu verwendende Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), femer (Metall)-Chelatoren (z.B. α-Hydroxyfettsäuren, Palmitinsäure, Phytinsäure, Lactoferrin), α-Hydroxysäuren (z.B. Zitronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), sowie Koniferylbenzoat des Benzoëharzes, Rutinsäure und deren Derivate, Ferulasäure und deren Derivate, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 10 Gew.-%, bezogen auf das Gesamtgewicht der Formulierung, zu wählen.

Es ist dem Fachmanne natürlich bekannt, daß anspruchsvolle kosmetische Zusammensetzungen zumeist nicht ohne die üblichen Hilfs- und Zusatzstoffe denkbar sind. Darunter zählen beispielsweise Konsistenzgeber, Füllstoffe, Parfum, Farbstoffe, Emulgatoren, zusätzliche Wirkstoffe wie Vitamine oder Proteine, Lichtschutzmittel, Stabilisatoren, Antioxidantien, Insektenrepellentien, Alkohol, Wasser, Salze, antimikrobiell, proteolytisch oder keratolytisch wirksame Substanzen usw.

Es ist besonders vorteilhaft, den erfindungsgemäß verwendeten Zubereitungen Puffersubstanzen zuzufügen. Insbesondere vorteilhaft ist, wenn die Zubereitungen auf pH-Werte von 5,5 oder kleiner abgepuffert werden.

Zur Anwendung werden die erfindungsgemäßen kosmetischen und dermatologischen Zubereitungen in der für Kosmetika üblichen Weise auf die Haut in ausreichender Menge aufgebracht.

Erfindungsgemäß verwendete kosmetische und dermatologische Zubereitungen können in verschiedenen Formen vorliegen, wie sie z.B. üblicherweise für diesen Typ von Zubereitungen eingesetzt werden. So können sie z.B. eine Lösung, eine Emulsion vom Typ Wasser-in-Öl (W/O) oder vom Typ Öl-in-Wasser (O/W), oder eine multiple Emulsionen, beispielsweise vom Typ Wasser-in-Öl-in-Wasser (W/O/W), ein Gel, einen festen Stift oder auch ein Aerosol darstellen.

Es ist auch möglich und vorteilhaft im Sinne der vorliegenden Erfindung, die erfindungsgemäßen Wirkstoffkombinationen in wäßrige Systeme bzw. Tensidzubereitungen zur Reinigung der Haut einzufügen.

Die erfindungsgemäß verwendeten kosmetischen und dermatologischen Zubereitungen können kosmetische Hilfsstoffe enthalten, wie sie üblicherweise in solchen Zubereitungen verwendet werden, z.B. Konservierungsmittel, Bakterizide, Parfüme, Substanzen zum Verhindern des Schäumens, Farbstoffe, Pigmente, die färbende Wirkung haben, Verdickungsmittel, oberflächenaktive Substanzen, Emulgatoren, weichmachende, anfeuchtende und/oder feuchthaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Sofern die erfindungsgemäß verwendete kosmetische oder dermatologische Zubereitung eine Lösung oder Lotion darstellt, können als Lösemittel verwendet werden:
- Wasser oder wäßrige Lösungen
- Öle, wie Triglyceride der Caprin- oder der Caprylsäure, vorzugsweise aber Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyloder -monobutylether, Diethylenglykolmonomethyl- oder -monoethylether und analoge Produkte.

Insbesondere werden Gemische der vorstehend genannten Lösungsmittel verwendet. Bei alkoholischen Lösungsmitteln kann Wasser ein weiterer Bestandteil sein.

Erfindungsgemäß verwendete Emulsionen sind vorteilhaft und enthalten z.B. die genannten Fette, Öle, Wachse und anderen Fettkörper, sowie Wasser und einen Emulgator, wie er üblicherweise für einen solchen Typ der Formulierung verwendet wird.

Erfindungsgemäß verwendete Zubereitungen können auch günstig als Gele vorliegen, die neben den erfindungsgemäßen Wirkstoffkombinationen und dafür üblicherweise verwendeten Lösemitteln, üblicherweise Alkohole niedriger C-Zahl, z.B. Ethanol, Isopropanol, 1,2-Propandiol, Glycerin und Wasser bzw. ein vorstehend genanntes Öl, dann noch organische Verdickungsmittel enthalten, z.B. Gummiarabikum, Xanthangummi, Natriumalginat, Cellulose-Derivate, vorzugsweise Methylcellulose, Hydroxymethylcellulose, Hydroxyethylcellulose, Hydroxypropylcellulose, Hydroxypropylmethylcellulose oder anorganische Verdickungsmittel, z.B. Siliciumdioxid und/oder Aluminiumsilikate wie beispielsweise Bentonite, oder ein Gemisch aus Polyethylenglykol und Polyethylenglykolstearat oder -distearat. Das oder die Verdickungsmittel sind im Gel z.B. in einer Menge zwischen 0,1 und 30 Gew.-%, bevorzugt zwischen 0,5 und 15 Gew.-%, enthalten.

Erfindungsgemäß verwendete feste Stifte enthalten z.B. natürliche oder synthetische Wachse, Fettalkohole oder Fettsäureester.

Es ist vorteilhaft, den erfindungsgemäß verwendeten Zubereitungen weitere gegen Akne wirksame Substanzen oder antientzündliche Wirkstoffe zuzugeben, beispielsweise Batylalkohol (α-Octadecylglycerylether), Selachylalkohol (α-9-Octadecenylglycerylether), Chimylalkohol (α-Hexadecylglycerylether) und/oder Bisabolol.

Bevorzugt können die Zubereitungen außerdem Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1 bis 6 Gew.-% beträgt, bezogen auf das Gesamtgewicht der Zubereitungen, um kosmetische und/oder dermatologische Zubereitungen zur Verfügung zu stellen, die die Haut vor dem gesamten Bereich der ultravioletten Strahlung schützen.

Enthalten die erfindungsgemäß verwendeten Zubereitungen UVB-Filtersubstanzen, können diese öllöslich oder wasserlöslich sein. Erfindungsgemäß vorteilhafte öllösliche UVB-Filter sind z.B.:
- 3-Benzylidencampher-Derivate, z.B. 3-(4-Methylbenzyliden)campher, 3-Benzylidencampher;
- 4-Aminobenzoësäure-Derivate, z.B. 4-(Dimethylamino)-benzoësäure(2-ethylhexyl)ester, 4-(Dimethylamino)benzoësäureamylester;
- Ester der Zimtsäure, z.B. 4-Methoxyzimtsäure(2-ethylhexyl)ester, 4-Methoxyzimtsäureisopentylester;
- Ester der Salicylsäure, z.B. Salicylsäure(2-ethylhexyl)ester, Salicylsäure(4-isopropylbenzyl)ester, Salicylsäurehomomenthylester,
- Derivate des Benzophenons, z.B. 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, z.B. 4-Methoxybenzalmalonsäuredi(2-ethylhexyl)ester, -2,4,6-Trianilino-(p-carbo-2'-ethyl-1'-hexyloxy) -1,3,5-triazin.

Vorteilhafte wasserlösliche UVB-Filter sind z.B.:
- Salze der 2-Phenylbenzimidazol-5-sulfonsäure wie ihr Natrium-, Kalium- oder ihr Triethanolammonium-Salz, sowie die Sulfonsäure selbst;
- Sulfonsäure-Derivate von Benzophenonen, wie z.B. 2-Hydroxy-4-methoxybenzophenon-5-sulfonsäure und ihre Salze;
- Sulfonsäure-Derivate des 3-Benzylidencamphers, wie z.B. 4-(2-Oxo-3-bornylidenmethyl)benzolsulfonsäure, 2-Methyl-5-(2-oxo-3-bornylidenmethyl)sulfonsäure und ihre Salze.

Enthalten die erfindungsgemäß verwendeten Zubereitungen UVA-Filtersubstanzen, können diese erfindungsgemäß vorteilhaft gewählt werden aus der Gruppe der Derivate des Dibenzoylmethans, z.B. 1-(4'-tert.Butylphenyl)-3-(4'-methoxyphenyl)propan-1,3-dion und 1-Phenyl-3-(4'-isopropylphenyl)propan-1,3-dion.

Die erfindungsgemäß verwendeten Zubereitungen können auch weitere anorganische Pigmente enthalten, die üblicherweise in der Kosmetik zum Schutze der Haut vor UV-Strahlen verwendet werden. Dabei handelt es sich um Oxide des Titans, Eisens, Zirkoniums, Siliciums, Mangans, Aluminiums, Cers und Mischungen bzw. Abwandlungen davon. Besonders bevorzugt handelt es sich um Pigmente auf der Basis von Titandioxid.

Sofern die kosmetische und/oder dermatologische Zubereitung eine Lösung oder Lotion darstellt, können als Lösungsmittel verwendet werden:
- Wasser oder wäßrige Lösungen
- Öle, wie Triglyceride der Caprin- oder der Caprylsäure, vorzugsweise aber Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder-monobutylether, Diethylenglykolmonomethyl- oder -monoethylether und analoge Produkte.

Insbesondere werden Gemische der vorstehend genannten Lösemittel verwendet. Bei alkoholischen Lösemitteln kann Wasser ein weiterer Bestandteil sein.

Ansonsten gelten für diese Gruppe an kosmetischen und/oder dermatologischen Zubereitungen die üblichen Anforderungen, die der Fachmann an solche Zubereitungen und deren Inhaltsstoffe stellt.

Die folgenden Beispiele wollen die vorliegende Erfindung erläutern, ohne daß eine Beschränkung auf den Gehalt der Beispiele beabsichtigt ist. Die Mengenangaben bedeuten stets, sofem nichts Anderes angegeben ist, Gewichts-%, bezogen auf das Gesamtgewicht der jeweiligen Zubereitungen.

### Beispiel 1

| Akne-Abdeck-Crème | |
|---|---|
| | Gew.-% |
| Ceteareth-20 | 2,00 |
| Butylenglycol | 5,00 |
| Myristylalkohol | 1,00 |
| Cetearylalkohol | 2,00 |
| Batylalkohol | 2,50 |
| Xanthangummi | 0,20 |
| Acrylat/C10-30-Alkylacrylatcopolymer | 0,50 |
| Carbomer . | 0,30 |
| Bisabolol | 0,50 |
| Panthenol | 2,80 |
| Titandioxid | 4,00 |
| Zinkoxid | 15,00 |
| Farbstoffe, Konservierungsmittel, Parfum | q.s. |
| Wasser | ad 100,00 |

### Beispiel 2

| Akne-Abdeck-Crème | |
|---|---|
| | Gew.-% |
| Steareth-20 | 2,00 |
| Butylenglycol | 5,00 |
| Myristylalkohol | 1,00 |
| Cetearylalkohol | 2,00 |
| Xanthangummi | 0,20 |
| Acrylat/C 10-30-Alkylacrylatcopolymer | 0,50 |
| Carbomer | 0,30 |
| Bisabolol | 0,50 |
| Panthenol | 2,80 |
| Titandioxid | 4,00 |
| Zinkoxid | 15,00 |
| Farbstoffe, Konservierungsmittel, Parfum | q.s. |
| Wasser | ad 100,00 |

## Patentansprüche

1. Verwendung
von 2,8 - 5,00 Gew.-% Panthenol
und
15 - 40,00 Gew.-% Zinkoxid
zur Herstellung eines kosmetischen und/oder dermatologischen mittels für die Behandlung von Akne und entzündeten Comedonen.

2. Verwendung nach Anspruch 1, **dadurch gekennzeichnet, daß** den Zubereitungen weitere antientzündliche Wirkstoffe zuzugeben werden, gewählt aus der Gruppe Batylalkohol, Selachylalkohol, Chimylalkohol und Bisabolol.

## Claims

1. Use of
2.8-5.00% by weight of panthenol
and
15-40.00% by weight of zinc oxide
for the preparation of a cosmetic and/or dermatological composition for the treatment of acne and inflamed comedones.

2. Use according to Claim 1, **characterized in that** further antiinflammatory active ingredients chosen from the group consisting of batyl alcohol, selachyl alcohol, chimyl alcohol and bisabolol, are added to the preparations.

## Revendications

1. Utilisation de 2,8 à 5% en poids de panthénol et de 15 à 40% en poids d'oxyde de zinc pour la préparation d'un agent cosmétique et/ou dermatologique pour le traitement de l'acné et de comédons enflammés.

2. Utilisation selon la revendication 1, **caractérisée en ce qu'**on ajoute aux préparations d'autres substances actives anti-inflammatoires, choisies parmi le groupe de l'alcool batylique, l'alcool sélachylique, l'alcool chimylique et le bisabolol.
